# EUROPEAN PATENT APPLICATION

(11) **EP 3 135 248 A1**
(43) Date of publication of application: **01.03.2017**
(21) Application number: 16185762.8
(22) Date of filing: 25.08.2016
(51) Int. Cl.: A61F 2/07

(54) **ENDOGRAFT WITH ONE OR MORE APERTURES**

(30) Priority: 26.08.2015 US 201514836385
(71) Applicant: Cook Medical Technologies LLC, Bloomington, IN 47404 (US)
(72) Inventor: EATON, Elizabeth A., Bloomington, IN 47401 (US); FRYE, Mark R., Bloomington, IN 47408 (US); HACKER, Allen E., Bloomington, IN 47404 (US)
(74) Representative: Williams Powell

(57) **Abstract**

The present embodiments describe an endograft (100) having one or more apertures, and methods for constructing the same. In one example, an endograft (100) comprises a proximal region (130) having a proximal opening (210), and a distal region (150) having first and second apertures (180) separated by a partition (240). A body portion (140) having a lumen (160) is disposed between the proximal region (130) and distal region (150), and has a central longitudinal axis. At least one of the first and second apertures (180) is oriented oblique to the central longitudinal axis.

## Description

The present invention relates generally to medical devices, and more particularly, to endografts used to treat a diseased vessel or region of vessels. In particular it relates to an endograft with one or more apertures.

The functional vessels of human and animal bodies, such as blood vessels and ducts, occasionally weaken or even rupture. For example, the aortic wall can weaken, resulting in an aneurysm. Upon further exposure to hemodynamic forces, such an aneurysm can rupture. One study found that in Western European and Australian men who are between 60 and 75 years of age, aortic aneurysms greater than 29 mm in diameter are found in 6.9% of the population, and those greater than 40 mm are present in 1.8% of the population.

One surgical intervention for weakened, aneurysmal, or ruptured vessels is EndoVascular Aneurysm Repair (EVAR) which involves accessing the diseased vasculature through peripheral vessels (e.g., iliac arteries) and deploying an endoluminal prosthesis such as a stent-graft or endograft. EVAR is less invasive than an open surgical repair, which involves opening the thorax and/or abdomen to sew in a replacement vessel analog (e.g., unsupported Dacron tube). In EVAR, the prosthesis may provide some or all of the functionality of the original, healthy vessel and/or preserve any remaining vascular integrity by replacing a length of the existing vessel wall that spans the site of vessel failure. A properly placed prosthesis excludes the diseased and/or aneurysmal portion of the vessel. For weakened or aneurysmal vessels, even a small leak ("endoleak") in or around the prosthesis may lead to the pressurization of the treated vessel which may aggravate the condition that the prosthesis was intended to treat. A prosthesis of this type can treat, for example, aneurysms of the abdominal aortic artery.

In general, delivery and deployment devices for endoluminal prostheses may include devices for retaining and releasing the prosthesis into the body lumen. For example, such a device may include a sheath for radially retaining the prosthesis in a compressed configuration. A pusher may be provided for pushing the sheath and the prosthesis into the body lumen and for delivering the device into a desired position. To deploy the prosthesis, the sheath may be withdrawn over the pusher and the prosthesis, thereby causing the prosthesis to become exposed and to expand into the body lumen.

The present invention seeks to provide an improved endograft.

The present embodiments describe an endograft having one or more apertures, and methods for constructing the same. In one embodiment, the endograft may comprise two apertures separated by a partition. Optionally, the apertures may be oriented at an oblique angle relative to a central longitudinal axis. Optionally, extension limbs may extend through the apertures. The endograft may include one or more fenestrations and/or an attachment stent to facilitate blood flow to specific anatomy.

In another example, the endograft may have at least one contralateral limb and at least one aperture. The aperture may be oriented at an oblique angle relative to a central longitudinal axis. Additionally, the aperture may be open or closed. In a related example, the endograft may comprise an extension limb extending through an open aperture.

In another example, the endografts may be constructed using a modular approach, with a main body and a tubular extension body, where the extension body may have one or more fenestrations.

According to an aspect of the present invention, there is provided an endograft, comprising: a proximal region having a proximal opening; a distal region having first and second apertures separated by a partition; a body portion having a lumen disposed between the proximal region and distal region, and having a central longitudinal axis, wherein at least one of the first and second apertures is oriented oblique to the central longitudinal axis.

Each of the first and second apertures may be oriented oblique to the central longitudinal axis.

The partition may be distal to the first and second apertures.

The first and second apertures may be on opposite sides of the distal region.

The partition and the body portion may be made of the same graft material.

The partition further comprises stitches disposed between the first and second apertures.

The endograft may further comprise an attachment stent coupled to the proximal region and extending proximally from the proximal opening.

The distal region may be coupled to the body portion via stitching.

The body portion may have one or more fenestrations.

The endograft may further comprise a first extension limb extending distally from the first aperture and sealably engaged to the first aperture, wherein the first extension limb has a proximal end, a distal end, and a lumen therebetween.

The first extension limb may sealingly engage an inner surface of the body portion above the distal region such that fluid flow is blocked through the second aperture.

A proximal end of the first limb extension may comprise a greater diameter than a distal end of the first limb extension in an expanded state.

The endograft may further comprise first and second extension limbs, each having a proximal end, a distal end, and a lumen therebetween, wherein the proximal end of the first extension limb sealably engages the first distal aperture, and the proximal end of the second extension limb sealably engages the second distal aperture.

The first and second oblique apertures may be each angled between 90-180 degrees, inclusive, from the central longitudinal axis.

According to another aspect of the present invention, there is provided an endograft for treating a section of a patient's vessel, comprising: at least one expandable stent; a graft material coupled to the at least one stent, to form a main body comprising: a proximal region having a proximal opening; a distal region having a contralateral limb with a proximal end, a distal end, and a lumen therebetween; a body portion between the proximal region and the contralateral limb, having a central longitudinal axis, a lumen, and at least one aperture oriented oblique to the central longitudinal axis.

The endograft may further comprise a first extension limb extending distally from the at least one aperture and sealably engaged to the at least one aperture, wherein the first extension limb has a proximal end, a distal end, and a lumen therebetween.

The first extension limb may sealingly engage an inner surface of the body portion above the distal region such that fluid flow is blocked through the contralateral limb.

A proximal end of the first limb extension may comprise a greater diameter than a distal end of the first limb extension in an expanded state.

The body portion may further comprise one or more fenestrations.

The at least one aperture may be angled between 90-180 degrees, inclusive, from the central longitudinal axis.

According to another aspect of the present invention, there is provided a method for manufacturing an endograft, comprising: attaching a graft material to at least one expandable wire stent to form a generally cylindrical primary body having a proximal region with a proximal opening, a distal region with a distal opening, and a lumen therebetween; attaching a secondary body to the primary body, wherein the secondary body has a central longitudinal axis, a proximal opening, at least one aperture oriented oblique to the central longitudinal axis, and an interior lumen extending between the proximal opening and the at least one aperture, and where attachment is via mating the distal region of the primary body to the proximal region of the secondary body such that the lumen of the primary and secondary bodies are in fluid communication.

Other systems, methods, features and advantages of the invention will be, or will become, apparent to one with skill in the art upon examination of the following Figures and detailed description. It is intended that all such additional systems, methods, features and advantages be within the scope of the invention, and be encompassed by the following claims.

Embodiments of the present invention are described below, by way of example only, with reference to the accompanying drawings, in which:
FIG. 1 is an anatomical view of an abdominal aortic aneurysm ("AAA");
FIGS. 2-3 are side and bottom views, respectively, of an embodiment of an endograft.
FIG. 4 is a side view of an endograft;
FIG. 5 is a side view of an endograft with a unilateral limb and an open oblique aperture;
FIG. 6 is a side view of an endograft with a unilateral limb and a closed oblique aperture;
FIG. 7 is a side view of an endograft with a unilateral limb, an open oblique aperture, and an extension limb extending through the aperture; and
FIGS. 8-9 are side views of embodiments of modular endografts, including a main body extension.

In the present application, the term "proximal" refers to a direction that is generally closest to the heart during a medical procedure, while the term "distal" refers to a direction that is furthest from the heart during a medical procedure.

The embodiments described below are in connection with systems and methods for the introduction and deployment of an implantable medical device in a vessel, such as endovascular prostheses, but could also be used for deploying a range of implantable medical devices including, but not limited to, stents, occlusion devices and the like.

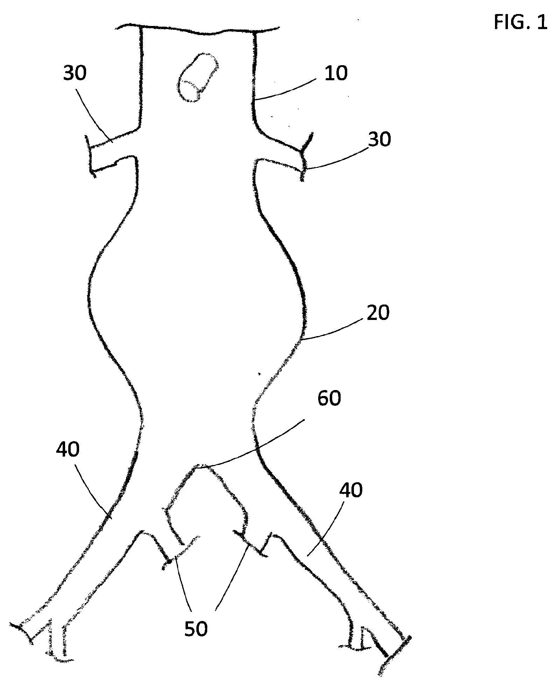

Referiing to FIG. 1, the aorta 10 is the largest artery in the human body. Over time, the walls of the aorta 10 may lose elasticity or otherwise weaken. Due to hemodynamic pressure, the vessel walls of the aorta 10 may expand in diameter, resulting in an aneurysm 20. FIG. 1 illustrates an example of an abdominal aortic aneurysm 20 ("AAA"), located infra to (below) the renal arteries 30 and supra to (above) the aortic bifurcation 60, external iliac arteries 40, and internal iliac arteries 50. An aneurysm 20 can increase the risk of a possibly fatal vessel rupture if the aneurysm expands and/or bursts. A common treatment for the aneurysm is to relieve the pressure on the aneurysm by redirecting blood flow through a stent graft or endograft.

Endografts may be implanted in the aorta 10, such that blood flowing past the aneurysm 20 flows through the endograft. Use of an endograft reduces pressure on the aneurysm 20 and can cause the aneurysm 20 to shrink in size. Endografts may incorporate self-expanding stents. The final shape, size, and position of the endograft in situ may also be modified through use of a balloon (e.g., CODA balloon catherer).

Endografts may be implanted in other arteries (not shown). For example, the renal arteries are not often aneurysmal, but may nonetheless be treated with covered stents in cases where there is insufficient healthy vessel length to use for sealing. In the case of a juxtarenal or thoraco-abdominal aortic aneurysm (TAAA), the aneurysm encompasses the ostia. To maintain a sealing zone, a minimal length (e.g., 4 mm) of native healthy vessel tissue is required below the renal arteries when placing a graft that will not include fenestrations or other accommodations.

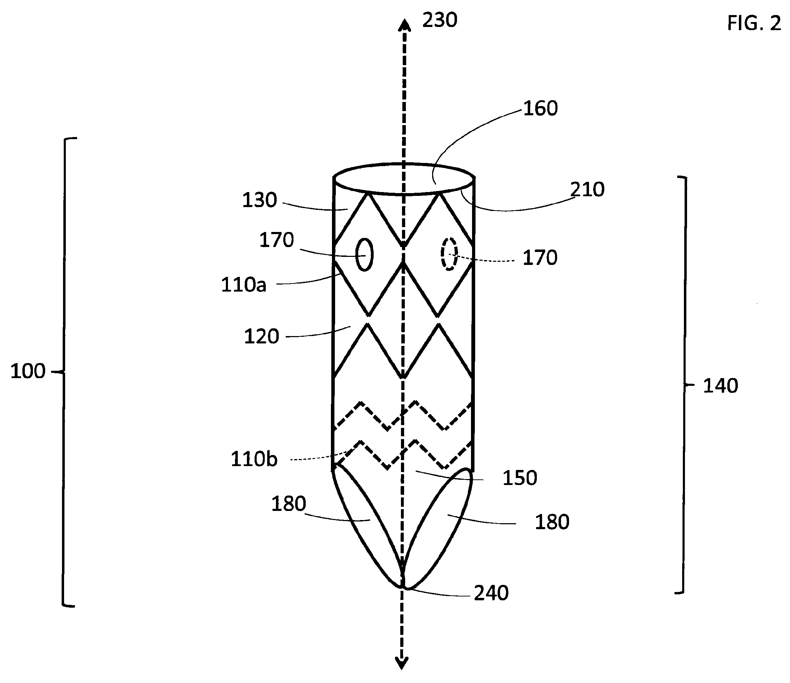

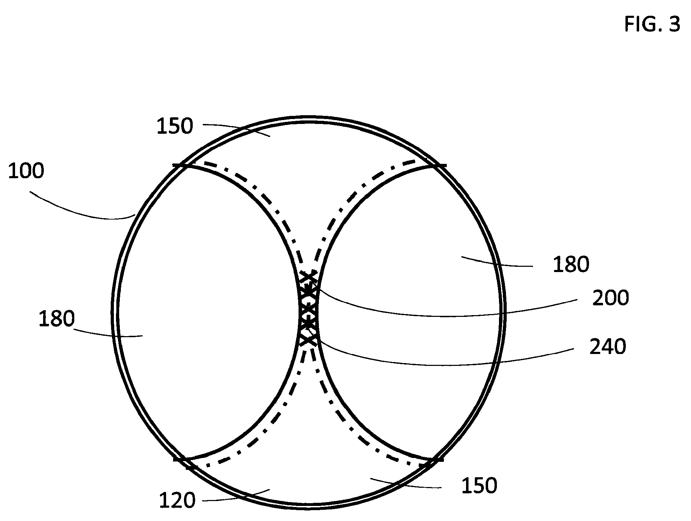

FIGS. 2-3 illustrate a side view and bottom view, respectively, of an embodiment of an endograft 100. Referring to FIGS. 2-3, an embodiment of the endograft 100 comprises an expandable support structure 110 and a graft material 120, including a main body 140 having a proximal region 130, a distal region 150, and a lumen 160 extending therebetween. The proximal region 130 may have a proximal opening 210, which may provide fluid access to the lumen 160 of the main body 140 and distal region 150. The main body 140 may be generally tubular in shape and have one or more fenestrations 170 in the graft material 120. The distal region 150 may have one or more apertures 180.

The support structure 110 of the endograft 100 may have any suitable stent pattern known in the art. The support structure 110 may be self-expanding or may expand under external pressures, for example from an inflatable balloon at the tip of a balloon catheter. One example of a stent pattern is the Z-stent or Gianturco stent design. Each Z-stent may include a series of substantially straight segments or struts interconnected by a series of bent segments or bends. The bent segments may include acute bends or apices. The Z-stents are arranged in a zigzag configuration in which the straight segments are set at angles relative to one another and are connected by the bent segments. Alternative stents may include, for example, annular or helical stents. The stents mentioned herein may be made from standard medical grade stainless steel. Other stents may be made from nitinol or other shape-memory materials.

Further, as shown in FIGS. 2-3, the main body 140 and distal region 150 may comprise at least one support structure 110, such as a stent. The support structure 110 may include a single, unitary structure or a plurality of independent structures. The support structure 110 and/or various portions thereof may be disposed on the inner surface and/or outer surface of the graft body 120. Multiple support structures 110 may be positioned at any point or points along a length of the endograft 100, as generally depicted in FIGS. 2-3.
In the current, non-limiting example, a plurality of external Z-stents 110a are disposed external to the graft material 120 at spaced-apart locations along the main body 140. Internal Z-stents 110b may also be disposed along portions of the main body 140, as shown in FIGS. 2-3. Given varying design configurations, some external Z-stents 110a may be replaced with internal Z-stents 110b, and vice versa.

The graft material 120 may be coupled to the external Z-stents 110a and internal Z-stents 110b by known methods, for example biocompatible stitching 200. The graft material 120 may be fabricated from any at least substantially biocompatible material including such materials as polyester fabrics, polytetrafluoroethylene (PTFE), expanded PTFE, and other synthetic materials known to those of skill in the art. In some embodiments in accordance with the technology, the graft material 120 may also include drug-eluting coatings or implants.

When deploying the endograft 100 into the lower aorta 10 in the region of aortic bifurcation 60 (or existing implant bifurcation (not shown) or new implant bifurcation (not shown)) , it may be desirable to provide at least one fenestration 170 in the main body 140 to avoid occluding the renal arteries 30 or the superior mesenteric artery. In the embodiment of FIGS. 2-3, the main body 140 has two substantially round or circular fenestrations 170 in the graft material 120 of the tubular wall of the endograft 100, providing fluid communication between the lumen 160 and each of the two renal arteries 30. Other numbers of fenestrations may also be used, as well as other opening types, for example, scallops and/or branches. The fenestrations 170 may be configured to house tubular extensions (not shown) that may extend into peripheral arteries, for example the renal arteries 30. As with all embodiments, radiopaque or MRI opaque markers may be used to define the periphery of the fenestrations 170. The fenestrations 170 may also be reinforced, for example, using biocompatible stitching 200.

The distal region 150 may have one or more apertures 180 that are substantially round or circular. The apertures 180 may be separated by a partition 240. The partition 240 may be formed by connecting portions of the distal region 150 via biocompatible stitching 200 or tailoring (not shown). The distal regions 150 and/or partition 240 may be contiguous with the graft material 120 of main body 140. Alternatively, the distal region 150, including the partition 240, may be formed from one piece that is connected to the main body 140, for example, via biocompatible stitching 200 or tailoring (not shown). Other manufacturing techniques can also be employed.

In certain embodiments (such as shown), the apertures 180 may have an oblique orientation relative to a central axis 230. The angle of the apertures 180 can be measured by measuring the angle between a normal vector to the plane of the aperture 180 and the central axis 230 of the endograft 100. For example, in the embodiment of FIGS. 2-3, the vector normal to the plane of the proximal opening 210 is parallel to the central axis 230, meaning zero degrees. The vector normal to the plane of the fenestrations 170 is substantially orthogonal to a central axis 230, meaning ninety degrees. The vector normal to the plane of the apertures 180 is greater than ninety degrees from the central axis 230, meaning it is an oblique angle. In the example of an AAA, oblique angles may facilitate bilateral blood flow to the external iliac arteries 40. In a preferred embodiment, the oblique angles may be within the range of 90-180 degrees relative to the central axis 230. In another example, the oblique angles may be 135 degrees relative to the central axis 230. The 135 degree angle is a theoretical optimum, though this may vary between 90-180 degrees (inclusive). When a physician accesses an aperture 180 intra-operatively, the specific oblique angle affects the "target size" for catheterization. For example, a catheter moving distally through the endograft 100, parallel to the central axis 230, and approaching an aperture 180 will have a greater "target" surface area if the oblique angle is 135 degrees versus ninety-one degrees. This "target size" may be critical to accessing the aperture 180, especially if the anatomy is crowded or diseased, or if other implanted structures are nearby.

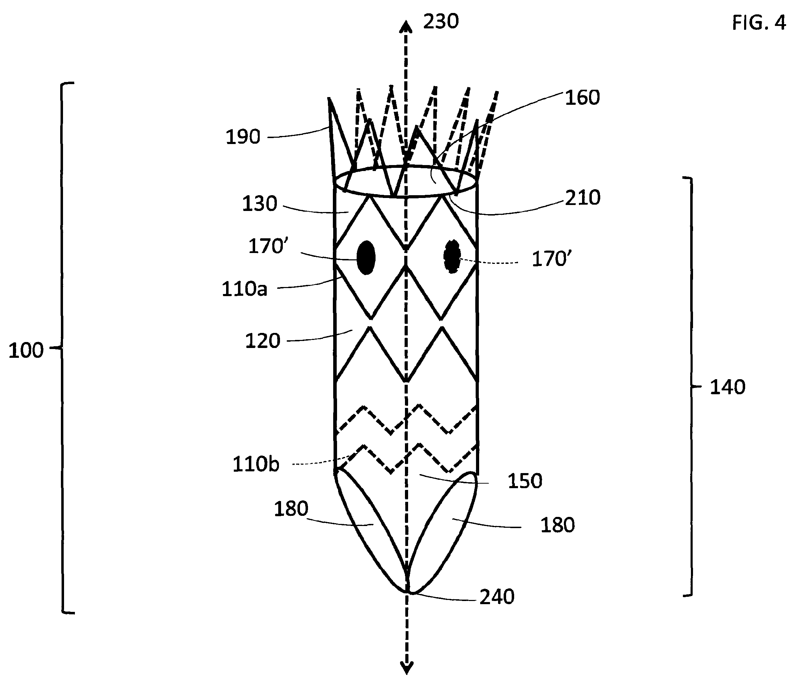

FIG. 4 illustrates a side view of an alternative embodiment of the endograft 100 having an attachment stent 190. The attachment stent 190 may have a distal end attached to the proximal region 130 of the main body 140, and a proximal end that extends proximally beyond the graft material 120, as shown in FIG. 4. Components of the attachment stent 190 may be distributed uniformly around the circumference of the proximal opening 210. The attachment stent 190 may have barbs or other fixation mechanisms (not shown) to secure the endograft 100 to the vessel walls. Similarly, the external Z-stents 110a and/or internal Z-stents 110b may also have barbs or other fixation mechanisms (not shown) to secure the endograft 100 to the vessel walls.

In the current, non-limiting example, the graft material 120 of the main body 140 may be disposed within the abdominal aorta 10 above an aortic bifurcation 60, such that the attachment stent 190 may span the renal arteries 30, whereby blood may flow through the struts of the attachment stent 190 and into the renal arteries 30. In such an embodiment, the fenestrations 170 may be replaced with closed fenestrations 170' (such as shown) or absent (not shown) to prevent fluid communication outside the endograft and to prevent endoleak. Alternatively, by varying the length of the main body 140, the fenestrations 170 may align with the renal arteries 30, as described above with respect to the embodiments shown in FIGS. 2-3, and the attachment stent 190 may provide structural support and prevent migration of the endograft 100. Varying the length of the main body 140 may also confer the advantage of selecting the best fit for the patient, including conforming to any existing implants already in place.

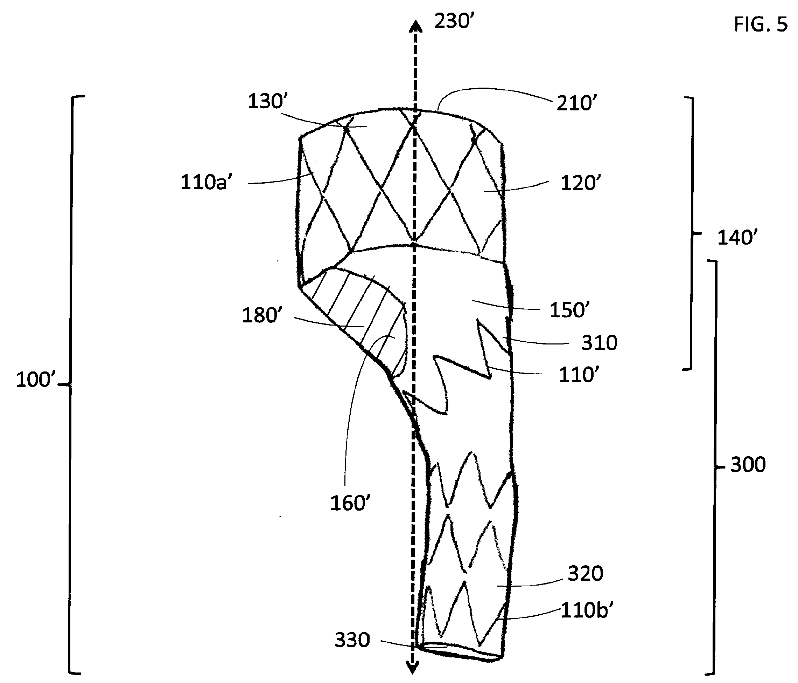

FIGS. 5-6 illustrate side views of another embodiment of an endograft 100'. Referring to FIG. 5, an embodiment of the endograft 100' comprises an expandable support structure 110' and a graft material 120', including a main body 140' having a proximal region 130', a distal region 150', and a lumen 160' extending therebetween. The endograft 100' may have a central longitudinal axis 230'. The proximal region 130' may have a proximal opening 210', which may provide fluid access to the lumen 160' of the main body 140' and distal region 150'. The main body 140' may be generally tubular in shape, and the distal region 150' may have one or more apertures 180'. Apertures 180' may have an oblique orientation relative to the central axis 230'.

The distal region 150' may transition into one or more contiguous limbs, for example, contralateral limb 300. The contralateral limb 300 may comprise proximal end 310, distal end 320, and a lumen 330 extending therebetween. The lumen 330 of the contralateral limb 300 may be in fluid communication with the lumen 160' of the endograft 100'.

The support structure 110' of the endograft 100' may have any suitable stent pattern known in the art. In the current, non-limiting example, the support structures 110' include external Z-stents 110a' on the proximal region 130' and internal Z-stents 110b' on the contralateral limb 300. The contralateral limb 300 may have multiple support structures 110' along the length from the proximal end 310 to the distal end 320. Given varying design configurations, some external Z-stents 110a' may be replaced with internal Z-stents 110b', and vice versa, or other support structures 110' known in the art.

The contralateral leg 300 may be made from a graft material 120' connected to the external Z-stents 110a' and internal Z-stents 110b' by known methods, for example biocompatible stitching or tailoring. The graft material 120' may be made from a substantially impermeable, biocompatible, and flexible material, as described above.

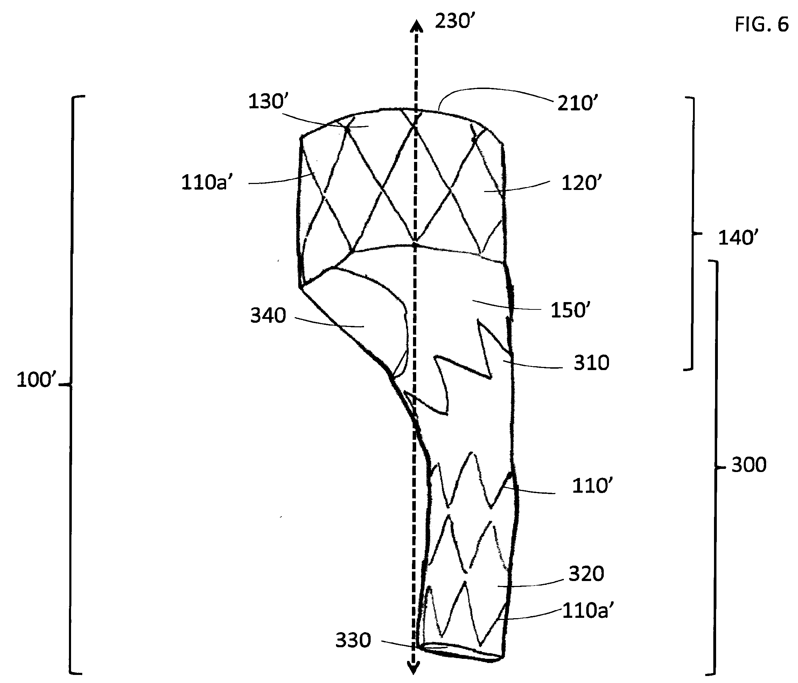

The aperture 180' may be either open or closed. In an open configuration, blood may flow bilaterally through both the contralateral limb 300 and the aperture 180', as shown in FIG. 5. In a closed configuration, a closure 340 may completely cover one or more apertures 180', as shown in FIG. 6. The closure 340 may be made of a graft material 120' and secured to the endograft 100' via biocompatible stitching or other method of securement, or simply may be integral with the graft material 120'. In the closed configuration, blood only flows unilaterally through the contralateral limb 300. This may be advantageous in certain patients. For example, in some cases, the clinician may be unable to gain bilateral access due to difficult anatomy, previously implanted devices, or extensive pathology including calcification or thrombosis. This is sometimes predictable pre-procedure, but not always. In general, it is better to maintain bilateral flow if at all possible. As such, physicians may try to cannulate both sides, and only opt for a unilateral approach (closed configuration) if they can't cannulate both sides. Alternatively, the patient may already be uni-iliac because of a prior femoral-femoral bypass, pathology, or the physician may be planning to sacrifice one side.

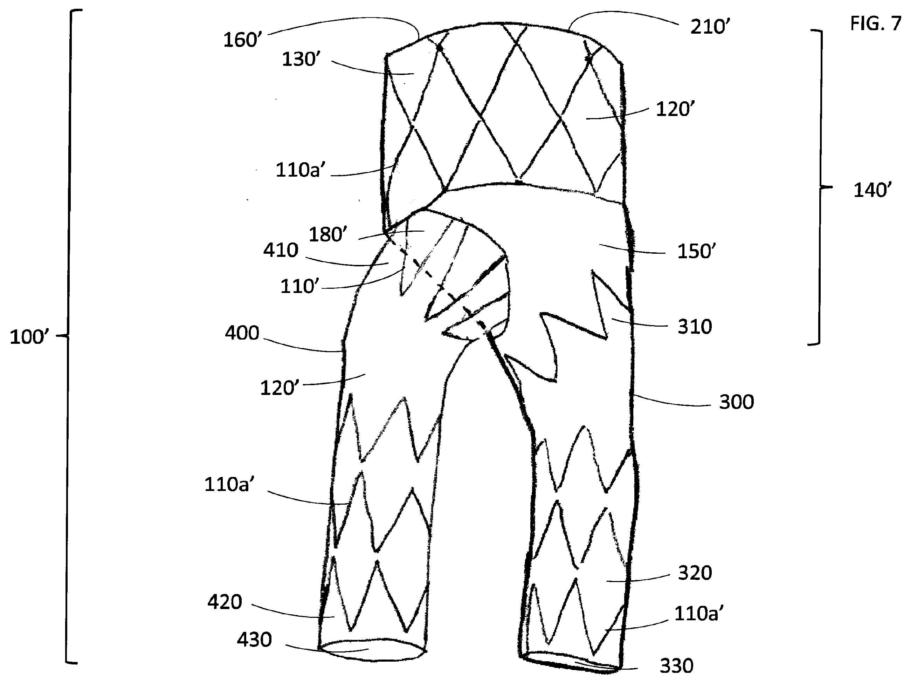

FIG. 7 illustrates a side view of the embodiment of the endograft 100' of FIG. 5, with the addition of an extension limb 400. Referring to FIG. 7, the extension limb 400 may comprise proximal end 410, distal end 420, and a lumen 430 extending therebetween. The lumen 430 of the extension limb 400 may be in fluid communication with the lumen 160' of the endograft 100', and the lumen 330 of the contralateral limb 300.

The extension limb 400 may have multiple support structures 110' along the length from the proximal end 410 to the distal end 420, for example external Z-stents 110a' . Given varying design configurations, some external Z-stents 110a' may be replaced with internal Z-stents 110b', and vice versa.

The extension limb 400 may be made from a graft material 120' connected to the external Z-stents 110a' and internal Z-stents 110b' by known methods, for example biocompatible stitching. The graft material 120' may be made from a substantially impermeable, biocompatible, and flexible material, as described above.

The proximal end 410 of extension limb 400 may sealingly engage with an aperture 180' of the endograft 100'. The seal may be formed via a compression fit, wherein the inner diameter of the aperture 180' is less than the outer diameter of the proximal end 410 of the extension limb 400. Additionally, the outer diameter of the distal end 420 of the extension limb 400 may be less than, equal to, or greater than the inner diameter of the aperture 180'. The distal end 420 of extension limb 400 may be compressed or otherwise configured to extend through the aperture 180' during implantation. The distal end 420 of extension limb 400 may sealingly engage with an inner surface of the surrounding blood vessel.

Additionally, the proximal end 410 of extension limb 400 may sealingly engage the endograft 100' proximal to the aperture 180', for example at the lumen 160' of the main body 140', and/or to the native vessel. For example, a bare attachment stent (not shown) may be connected proximal to the proximal end 410 of the extension limb 400 and deployed to extend into the lumen 160' proximal to the aperture 180'. The bare attachment stent may circumferentially engage the graft material 120' of the lumen 160' to secure the position of the leg without interrupting bilateral flow (since flow would be maintained through the wires of the bare attachment stent). A seal stent may also be used to maintain an adequate seal with the endograft 100' without interrupting flow.

In another example, the attachment stent may instead be covered either partially or entirely with a graft material. In one example, the attachment stent may be covered with a graft material to engage the lumen 160' of main body 140', wherein the graft material may also have an aperture (not shown) to provide fluid communication with the lumen 330 of contralateral leg 300. Some bare wire(s) of the attachment stent may span portions of the aperture, and may be sewn into a fixed position or allowed to open freely through the aperture. Alternatively, portions of the bare attachment stent may be removed, for example, portions of the stent structure spanning the aperture.

In another embodiment (not shown), multiple extension limbs 400 may sealingly engage with multiple apertures 180 or apertures 180'. For example, the embodiments of FIGS. 2-4 have two apertures 180, each of which could engage a proximal end 410 of an extension limb. The distal ends 420 of both extension limbs 400 would sealingly engage with inner surfaces of the surrounding blood vessels. This bilateral embodiment may be advantageous where the treatment site is near a bifurcation or other branching of blood vessels. Alternatively, multiple extension limbs 400 may sealingly engage the endograft 100 proximal to the apertures 180, for example at the proximal region 130.

The embodiments described herein provide two non-limiting examples of endografts that are suitable for treating an array of medical conditions, and may be especially suited for treating an abdominal aortic aneurysm 20 at or slightly above the aortic bifurcation 60. Alternatively, the bifurcation may within an existing endograft, for example one that was newly or previously implanted. As will be appreciated, the main body 140 (or 140') may be positioned in the abdominal aorta 10 slightly above the aortic bifurcation 60, while the ipsilateral extension limb 400 may extend into one external iliac artery 40 and the contralateral limb 300 may extend into, or be positioned slightly above, the opposing external iliac artery 40, depending on length. These embodiments (and related embodiments) may be beneficial where the length between the visceral vessels (e.g., renal arteries) and the bifurcation may be extremely short, making it difficult to implant endografts with longer body lengths while maintaining access to the visceral vessels and simultaneously engaging healthy vessel walls.

Various additional modular components may be provided for the endograft 100 (or 100'), for example, additional extension limbs may be configured to overlap with the distal end 320 of the contralateral limb 300. Such an extension limb would have a proximal end that sealingly overlaps with the contralateral limb 300, and a distal end that sealingly engages an inner surface of the external iliac artery 40.

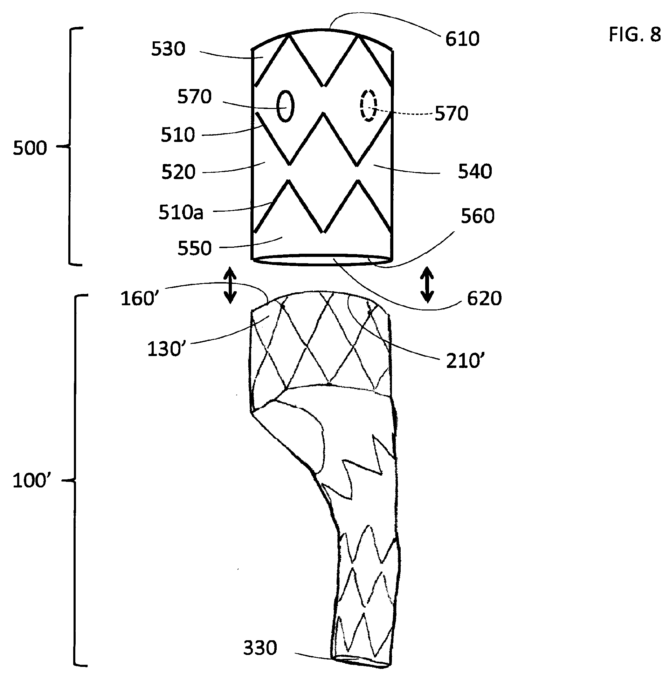

In another modular design, embodiments of endograft 100' (or 100) may be constructed using a multi-piece construction to form a longer main body. As shown in FIG. 8, a tubular main body extension 500 may be coupled to the proximal region 130' (or 130) of endograft 100' (or 100), for example, via biocompatible stitching or tailoring. The tubular main body extension 500 may be made from a graft material 520 attached to one or more support structures 510, which may be external Z-stents 510a or internal Z-stents (not shown), as described above. The tubular main body extension 500 may have a main body 540, including a proximal region 530, a distal region 550, and a lumen 560 extending therebetween. The main body 540 may have one or more fenestrations 570, and such fenestrations may be configured to align with specific anatomy, for example, the renal arteries 30. Alternatively, the tubular main body extension 500 may have an attachment stent (not shown) to provide access to the renal arteries 30.

The proximal region 530 may include a proximal opening 610 and the distal region 550 may include a distal opening 620. In the attached configuration, the lumen 560 of the tubular main body extension 500 may be in fluid communication with the proximal opening 610, the distal opening 620, the main body lumen 160' (or 160), the contralateral limb lumen 330, and any extension limb lumen (not shown).

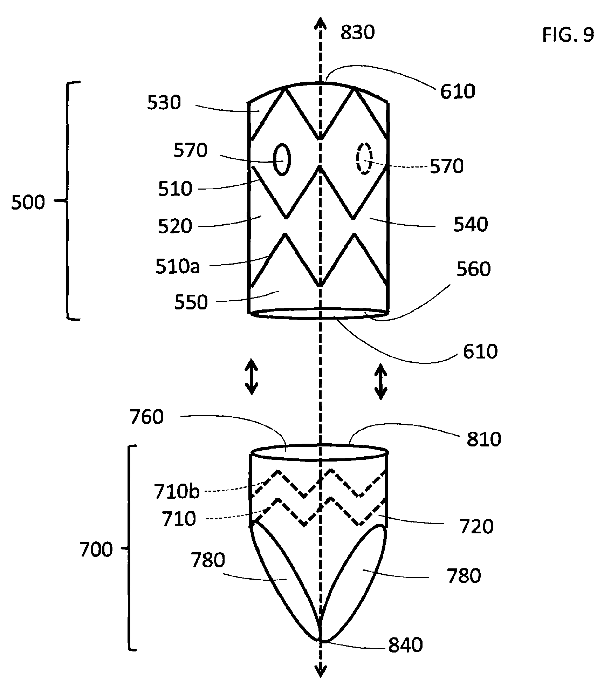

Although FIG. 8 illustrates this embodiment with endograft 100' of FIG. 6, the tubular main body extension 500 may be attached to any endograft 100 or 100', including but not limited to those shown in FIGS. 2-7. Referring now to the embodiment of FIG. 9, the tubular main body extension 500 may be attached to main body 700. Main body 700 may be made from a graft material 720 coupled to one or more support structures 710, which may be internal Z-stents 710b or external Z-stents (not shown), as described above. The main body 700 may have one or more apertures 780, a proximal opening 810, and a lumen 760 extending therebetween. The one or more apertures 780 may be separated by a partition 840. The partition 840 may be distal to the apertures 780. The one or more apertures may be oriented at an oblique angle relative to a central longitudinal axis 830, as described above. The main body 700 and tubular main body extension 500 may be attached by conventional methods, for example, biocompatible stitching or tailoring (not shown), such that the lumen 760 and lumen 560 are in fluid communication.

The modular design illustrated in FIGS. 8-9 may reduce manufacturing complexity and/or cost. Although a two-piece construction is illustrated, the final device may also be constructed from a single unit or more than two units.

Another benefit to the modular design is customization, both for clock position (rotational orientation about the central longitudinal axis) as well as length. This is advantageous because the manufacture of custom grafts (common for patients requiring fenestrations) may take a long time, delaying procedures that may be better performed as soon as possible. It also allows the physician to evaluate and customize on-the-fly during a procedure, by using different off-the-shelf components as the procedure advances in the operating room. The time between pre-operation imaging/scans and the procedure itself, as well as changes due to the influence of components placed, contribute to the need to be flexible intra-procedurally. Customization may be possible even where the fenestrations are included in the same piece as the apertures, since the physician could use extension limbs to curve around to openings in a bifurcation (e.g., aortic bifurcation or graft bifurcation). Furthermore, with multiple pieces where the fenestrations and apertures are initially in separate components, customization is even easier and more customization options are available (e.g., clock position, body length).

While references to treatment of an aneurysm 20 at or near the aortic bifurcation 60 may be explained as one example, it will be appreciated that endografts 100 and 100' can be positioned at other bodily locations to treat aneurysms 20 or other conditions, using the system and methods described herein.

One additional use of the embodiments described herein is for use as a secondary repair device. A patient may have a primary device previously implanted to treat an aneurysm or other condition. Over time, the effectiveness of the primary device may decrease, for example due to technical overreach, device failure, endoleaks, or disease progression. However, moving and replacing such a primary device may involve a high risk procedure. This is especially relevant as patients live longer (and live longer with endografts). As patient populations age, they tend to present with more and more challenges to open surgical procedures (co-morbidities, changes in anatomy, etc.). The only way of moving an existing device is (generally) through open repair, and many such patients may be poor candidates for open surgery. A repair option that accounts for repairs over time and that is compatible with existing implants may be a viable alternative.

The embodiments described herein may be advantageously used to repair such primary devices. The expandable support structure 110 (or 110') may fit inside primary devices of many sizes, making it nearly "universal." To augment or repair anatomy proximal to the primary graft and near a crucial juncture such as the renal arteries 30, the embodiments of FIGS. 2-4 (and related embodiments) having one or more fenestrations will be advantageous. To augment or repair anatomy distal to a primary device, then the embodiments of FIGS. 5-7 (and related embodiments) having contralateral limbs 300 and/or extension limbs 400 may be advantageous. One advantage is that the present embodiments may function as repair device because the limbs will be held in place by the repair endograft 100 (or 100'), regardless of whether the primary device is configured for extension limbs 400.

While various embodiments of the invention have been described, the invention is not to be restricted except in light of the attached claims. Moreover, the advantages described herein are not necessarily the only advantages of the invention and it is not necessarily expected that every embodiment of the invention will achieve all of the advantages described.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

The disclosures in United States patent application number 14/836,385, from which this application claims priority, and in the abstract accompanying this application are incorporated herein by reference.

## Claims

1. An endograft (100), comprising:
a proximal region (130) having a proximal opening (210);
a distal region (150) having first and second apertures (180) separated by a partition (240);
a body portion (140) having a lumen (160) disposed between the proximal region and distal region, and having a central longitudinal axis,
wherein at least one of the first and second apertures is oriented oblique to the central longitudinal axis.

2. An endograft (100) as claimed in claim 1, wherein the partition (240) is distal to the first and second apertures (180).

3. An endograft (100) as claimed in claim 1 or 2, wherein the first and second apertures (180) are on opposite sides of the distal region (150).

4. An endograft (100) as claimed in claim 1, 2 or 3 wherein the partition (240) and the body portion (140) are made of the same graft material.

5. An endograft (100) as claimed in any preceding claim, wherein the partition (240) comprises stitches disposed between the first and second apertures (180).

6. An endograft (100) as claimed in any preceding claim, comprising an attachment stent coupled to the proximal region (130) and extending proximally from the proximal opening (210).

7. An endograft (100) as claimed in any preceding claim, wherein the distal region (150) is coupled to the body portion (140) via stitching.

8. An endograft (100) as claimed in any preceding claim, further comprising first and second extension limbs, each having a proximal end, a distal end, and a lumen therebetween, wherein the proximal end of the first extension limb sealably engages the first distal aperture (180), and the proximal end of the second extension limb sealably engages the second distal aperture (180).

9. An endograft (100) as claimed in any preceding claim, wherein each of the first and second apertures (180) is oriented oblique to the central longitudinal axis.

10. An endograft (100) for treating a section of a patient's vessel, comprising:
at least one expandable stent;
a graft material coupled to the at least one stent, to form a main body comprising,
a proximal region (130) having a proximal opening (210);
a distal region (150) having a contralateral limb with a proximal end, a distal end, and a lumen therebetween;
a body portion (140) between the proximal region and the contralateral limb, having a central longitudinal axis, a lumen, and at least one aperture oriented oblique to the central longitudinal axis.

11. An endograft (100) as claimed in claim 9 or 10, wherein the first and second oblique apertures (180) or the at least one aperture are each angled between 90-180 degrees, inclusive, from the central longitudinal axis.

12. An endograft (100) as claimed in any preceding claim, comprising a first extension limb extending distally from the first aperture (180) or the at least one aperture and sealably engaged to the first aperture or the at least one aperture, wherein the first extension limb has a proximal end, a distal end, and a lumen therebetween.

13. An endograft (100) as claimed in claim 12, wherein the first extension limb sealingly engages an inner surface of the body portion (140) above the distal region (150) such that fluid flow is blocked through the second aperture (180) or the contralateral limb, optionally wherein a proximal end of the first limb extension comprises a greater diameter than a distal end of the first limb extension in an expanded state.

14. An endograft (100) as claimed in any preceding claim, wherein the body portion (140) has one or more fenestrations.

15. A method for manufacturing an endograft, comprising:
attaching a graft material to at least one expandable wire stent to form a generally cylindrical primary body having a proximal region with a proximal opening, a distal region with a distal opening, and a lumen therebetween;
attaching a secondary body to the primary body,
wherein the secondary body has a central longitudinal axis, a proximal opening, at least one aperture oriented oblique to the central longitudinal axis, and an interior lumen extending between the proximal opening and the at least one aperture,
and where attachment is via mating the distal region of the primary body to the proximal region of the secondary body such that the lumen of the primary and secondary bodies are in fluid communication.
